# EUROPEAN PATENT APPLICATION

(11) **EP 2 745 808 A1**
(43) Date of publication of application: **25.06.2014**
(21) Application number: 12823435.8
(22) Date of filing: 15.08.2012
(51) Int. Cl.: A61F 2/82, A61L 31/18

(54) **INTRAVASCULAR STENT WITH IMPROVED VISUALIZATION PERFORMANCE AND METHOD FOR ENHANCING THE VISUALIZATION PERFORMANCE OF INTRAVASCULAR STENT**

(30) Priority: 15.08.2011 CN 201110234883
(71) Applicant: Shanghai MicroPort Medical (Group) Co., Ltd., Shanghai 201203 (CN)
(72) Inventor: WANAG, Sen, Shanghai 201203 (CN); XIE, Zhiyong, Shanghai 201203 (CN); LV, Jian, Shanghai 201203 (CN); XU, Xiaohong, Shanghai 201203 (CN); JIN, Qiaorong, Shanghai 201203 (CN); LUO, Qiyi, Shanghai 201203 (CN)
(74) Representative: Dantz, Jan Henning
(86) International application number: PCT/CN2012/080136
(87) International publication number: WO 2013/023583

(57) **Abstract**

An intravascular stent, especially an intracranial vascular stent, wherein a stent strut (1) of the stent is completely wound with at least one layer of wire (2) made from a visualizable material. The stent can ensure accurate positioning under medical imaging equipment during and after a surgery. In addition, since the wound visualizable wire (2) is located at a position on the stent subjected to little or no stress, the expansion and the stress distribution of the stent is not affected, so that the service life of the stent will not be shortened, nor will the expected effect of the stent in use be influenced. Also disclosed is a method for enhancing a visualization performance of an intravascular stent.

## Description

### Technical Field

The invention relates to an intravascular stent, especially an intracranial vascular stent, with an improved visualization performance. The invention further relates to a method for enhancing a visualization performance of an intravascular stent.

### Background Art

Brain vascular aneurysm and vascular arterial stenosis are diseases with a high death rate and a high disability rate. Intravascular stents are widely used as implants for treating such vascular diseases. Generally, an intravascular stent is delivered to a pathological location through a delivery system, and then the blood vessel is reconstructed through the expansion of the stent, thereby treating the aneurysm or the arterial stenosis. Thus, endovascular interventional treatment by means of an intravascular stent gradually becomes the primary method for treating such vascular diseases. The treatment has the following advantages: fewer traumas, less complications, increased safety, less pain suffered by patients, easy acceptability, short hospital length of stay and tolerability by elderly patients who are seriously ill.

The visualization performance of an intravascular stent under an X-ray machine or other medical imaging equipment is one important characteristic among the numerous performances of the stent, and plays a crucial role in terms of the clinical effect during and after the surgery. During the surgery, the doctor needs to accurately position and release the stent by means of the visualization performance of the intravascular stent to ensure that the stent can be accurately implanted to the pathological location. After the surgery, follow-up is required to ensure that the stent will not be displaced so as to cause new thrombus.

Current intravascular stents are generally made from 316L stainless steel, a cobalt-chromium alloy, a nickel-titanium alloy, a magnesium alloy, a polymeric material or the like. With respect to an intracranial vascular stent, due to the special physiologically anatomic structure of intracranial vessels (e.g., because of the existence of the skull), the visualization performance of the intracranial vascular stent is affected. In addition, the requirement on the flexibility of the intracranial vascular stent makes the metal coverage of the metal framework of the intracranial vascular stent less than that of a coronary artery stent, which is another factor that results in a comparatively poor visualization performance of the intracranial vascular stent. If the visualization performance is increased only depending on the stent material itself, the object of enhancing the visualization performance can only be achieved by increasing the size of the structure of the stent, which, however, means more metal implantation. From a clinical perspective, this will reduce biocompatibility and increase probability of thrombosis. Meanwhile, the stent will become harder due to the increase in metal material, and thus hinder the stent passing through a blood vessel which is complex and has a small diameter.

One manner for enhancing the visualization performance in the prior art is to improve the visualization performance of a stent by increasing the visualization performance of the proximal and distal ends of the stent, which is generally achieved by fixing a visualizable tube or visualizable material to the two ends of the stent by adopting a certain process. However, such a design has the following defects: firstly, only two end points, i.e., distal and proximal end points, of the stent, rather than the entire stent can be displayed, and if the process is not stable, the visualizable tube or visualizable material may tend to fall off; in addition, with such a design, the outer diameter of the end portion of the stent is large, which is unfavorable for assembling the stent in terms of the process and affects the flexibility of the stent.

In the prior art, some examples are disclosed for enhancing the visualization performance of stents. Chinese patent application CN200880105999.9 discloses a stent with radiopaque markers, wherein the radiopaque markers are provided onto a ring at the end portions of the stent. U.S. patent application US2004044399A1 discloses a stent with radiopaque interconnected elements, wherein the interconnected elements are encapsulated by a polymeric material in a coil-like configuration and are interconnected at the proximal and distal ends of the stent. European patent application EP1362564A1 discloses an intravascular stent, comprising anchor members, which are comprised of a longitudinal leg having a radiopaque coil disposed about the leg. U.S. patent application US2004078071A1 discloses a stent, which also has anchor elements that are formed by winding a radiopaque coil onto a threaded portion of a strut of the stent. U.S. patent application US 6,334,871 B1 discloses a stent, which also has radiopaque markers. Chinese patent application CN200480044170.4 discloses a radiopaque bioabsorbable polymer stent, wherein the polymer contains sufficient halogen atoms to make the stent have a radiopaque performance. U.S. patent application US2010152837A1 discloses an intravascular stent, comprising a radiopaque inner core and a polymer layer disposed about the radiopaque inner core. U.S. patent application US2007185564A1 discloses a stent, wherein the stent material itself has a radiopaque component.

All the stents in the above documents are made from materials with a good visualization performance, and the method for enhancing the visualization of the stents is to wind a part of the respective stents with a visualizable wire or wrap the visualizable material with the stent material. In this manner, the stents can be visualized under the Digital Subtraction Angiography (DSA) without affecting the stents' performance. However, the stents which are wound with the visualizable wire in the prior art can be only partially visualized and cannot be entirely visualized. Also, with respect to the stents in the prior art where the visualizable material is wrapped in the stent strut, due to the restriction by the width of the stent strut, the amount of the contained visualizable material is limited, so that the visualization performance is restricted. Further, the manners disclosed in the above documents, in which the visualization performance is improved, complicate the manufacture of the stents.

### Summary of Invention

With respect to these defects existing in the prior art, the invention provides an improved visualization design for an intravascular stent, especially an intracranial vascular stent.

According to the invention, an intravascular stent is provided, wherein a stent strut of the stent is completely wound with at least one layer of wire made from a visualizable material. The invention can ensure accurate positioning of the stent under medical imaging equipment during and after a surgery. Since the wound visualizable wire is located at a position on the stent subjected to no stress or to very little stress, the expansion and the stress distribution of the stent is not affected, so that the service life of the stent will not be shortened, nor will the expected effect of the stent in use be influenced.

According to the invention, the stent strut is wound with a visualizable wire. The wire is arranged independently and at intervals or arranged continuously and closely in the axial direction of the stent strut. The winding of the wire can be achieved by spirally winding the stent strut with an entire wire, e.g., in the form of a spring coil. When the wire is arranged continuously and closely in the axial direction of the stent strut (strut), the wire can be wound to form a multilayer stack. The wound wire can also be arranged at intervals and wound to form a multilayer stack. The wire, when wound as a multilayer stack, can be achieved in the following manner: the stent strut is firstly spirally wound with one wire, and the previously wound wire is further spirally wound with another wire.

According to the invention, the visualizable material is made from a material having a good X-ray opaque performance, a strong corrosion resistance and a good biocompatibility, and can consist of materials such as gold, platinum, tantalum, osmium, rhenium, tungsten, iridium and rhodium or alloys of the above materials.

According to the invention, the stent can be a laser cut stent, a braided stent, a metal stent or a polymeric material stent. The stent can be implanted in place by expanding a balloon, or can be implanted in place by means of self expansion.

The invention further provides a method for enhancing a visualization performance of an intravascular stent, wherein the stent strut of the stent is completely wound with at least one layer of wire made from a visualizable material.

The design for enhancing a visualization performance of an intravascular stent of the invention has the following advantages: the design is applicable to various intravascular stents; the stent has a strong flexibility in use; the entire stent can be displayed under DSA; although the stent strut is completely wound with the wire over the entire stent, the wire acts on the radial direction of the stent strut, and the visualization effect can be further improved by a multilayer winding without affecting the physical performance of the stent; the flexibility or the like of the stent can be kept; the wire is applied to the stent by adopting the simple winding process.

### Brief Description of the Drawings

By way of the detailed descriptions of the embodiments of the invention below with reference to the figures, the characteristics and advantages of the invention will be obvious. In the figures,

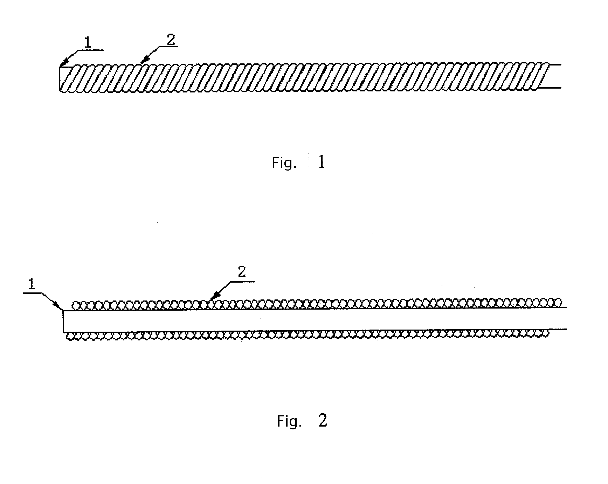

Fig. 1 and Fig. 2 are schematic diagrams where the wire is arranged continuously and closely on the stent strut in the axial direction.

### Detailed Description

Figs. 1 and 2 show one embodiment of the invention, and show how a wire strut 1 from which a braided stent is formed, is completely wound with a visualizable wire 2, the wire 1 being one of the wires used for the braided stent. As shown in Figs. 1 and 2, one layer of visualizable wire 2 is evenly wound onto the wire 1, wherein the visualizable wire 2 is arranged continuously and closely in the axial direction of the wire 1, with substantially no gap therebetween. Certainly, the visualizable wire can be also arranged at intervals. The slope formed by winding the wire 1 with the visualizable wire 2 is automatically determined according to the diameters of the visualizable wire 2 and the wire 1. The visualizable wire 2 is kept unchanged in terms of the diameter and is not deformed in the winding process.

The wire 1 is a round wire, and has a diameter of 0.02 ∼ 0.1mm. The visualizable wire 2 is a round wire, and has a diameter of 0.01 ∼ 0.08mm.

According to another embodiment of the invention, a stent strut of a laser cut stent is completely wound with a visualizable wire.

According to a further embodiment of the invention, a stent strut is completely wound with multiple layers of visualizable wire to further enhance a visualization performance of an intravascular stent. This is done by firstly spirally winding the stent strut with one wire, and then spirally winding the previously wound wire with another wire. Similarly, the wound wire can be arranged at intervals or arranged continuously and closely.

The intravascular stent with an improved visualization performance of the invention is convenient in machining, is simple in the manufacturing process, its visualizable wire is not likely to fall off, is good in the visualization performance, and does not have a high requirement on the sizes of the stent and the visualizable wire.

Those skilled in the art can understand that the above descriptions are merely exemplary. Those skilled in the art can make various modifications and variants to the invention without departing from the concept and scope of the invention.

## Claims

1. An intravascular stent, comprising a stent strut, **characterized in that** a stent strut (1) of the stent is completely wound with at least one layer of wire (2) made from a visualizable material.

2. The intravascular stent according to claim 1, wherein the wire (2) is arranged independently and at intervals or arranged continuously and closely in the axial direction of the stent strut (1).

3. The intravascular stent according to claim 2, wherein the stent strut (1) is firstly spirally wound with one layer of wire (2), and the previously wound one layer of wire (2) is further spirally wound with another layer of wire (2).

4. The intravascular stent according to claim 1, wherein the visualizable material is a material having a good X-ray opaque performance, a strong corrosion resistance and a good biocompatibility.

5. The intravascular stent according to claim 4, wherein the visualizable material includes gold, platinum, tantalum, osmium, rhenium, tungsten, iridium, rhodium or alloys of the above materials.

6. The intravascular stent according to claim 1, wherein the intravascular stent includes an intracranial vascular stent.

7. The intravascular stent according to any one of claims 1-6, wherein the intravascular stent is a laser cut stent, a braided stent, a metal stent or a polymeric material stent.

8. A method for enhancing a visualization performance of an intravascular stent, **characterized by** winding a stent strut (1) of the stent completely with at least one layer of wire (2) made from a visualizable material.

9. The method according to claim 8, wherein the wire (2) is arranged independently and at intervals or arranged continuously and closely in the axial direction of the stent strut (1).

10. The method according to claim 9, wherein the stent strut (1) is firstly spirally wound with one layer of wire (2), and the previously wound one layer of wire (2) is further spirally wound with another layer of wire (2).
